(19) Europäisches Patentamt
European Patent Office
Office européen des brevets

(11) Veröffentlichungsnummer : **0 047 912**
**B1**

(12) # EUROPÄISCHE PATENTSCHRIFT

(45) Veröffentlichungstag der Patentschrift : **28.12.83**

(51) Int. Cl.$^3$ : **C 07 D209/48**

(21) Anmeldenummer : **81106791.7**

(22) Anmeldetag : **31.08.81**

(54) **Verfahren zur Herstellung von N-(Cyclohexylthio)-phthalimid.**

(30) Priorität : **12.09.80 DE 3034397**

(43) Veröffentlichungstag der Anmeldung : **24.03.82 Patentblatt 82/12**

(45) Bekanntmachung des Hinweises auf die Patenterteilung : **28.12.83 Patentblatt 83/52**

(84) Benannte Vertragsstaaten : **BE CH DE FR GB IT LI NL**

(56) Entgegenhaltungen :
**AT-B- 226 680**
**US-A- 3 586 696**
**US-A- 3 840 555**
**HOUBEN-WEYL "Methoden der organischen Chemie" 4. Auflage, Band IX, 1955, GEORG THIEME VERLAG, Stuttgart, Seiten 275 to 277**
**The Journal of Organic Chemistry, Vol. 34, Seiten 51-55 (Jan.1969)**

(73) Patentinhaber : **BAYER AG**
**Zentralbereich Patente, Marken und Lizenzen**
**D-5090 Leverkusen 1, Bayerwerk (DE)**

(72) Erfinder : **Wassen, Jürgen, Dr.**
**Dürscheider Weg 4**
**D-5090 Leverkusen 3 (DE)**
Erfinder : **Königshofen, Heinrich, Dr.**
**Am Mühlenberg 26**
**D-5060 Bergisch-Gladbach 2 (DE)**

**EP 0 047 912 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von N-(Cyclohexylthio)-phthalimid

Die Erfindung betrifft ein Verfahren zur Herstellung von N-(Cyclohexylthio)-phthalimid durch Umsetzung von Cyclohexylsulfenylchlorid mit Phthalimid in Gegenwart von wäßrigen Alkali- oder Erdalkalilösungen.

Zur Herstellung von N-(Cyclohexylthio)-phthalimid sind bereits mehrere Verfahren bekannt. So beschreiben Sigeru Torii et al. in J. Org. Chem. 44, 1554-57 (1979) die elektrolytische Synthese aus Phthalimid und Dicyclohexyldisulfid. Dieses Verfahren ist jedoch großtechnisch nicht zu realisieren.

Durch die Arbeiten von O.A. Ermakov et al. in Zh. Org. Khim. *14,* 1299-1301 (1978) und Zh. Org. Khim. *12,* 237 (1976) ist weiterhin bekannt, daß ausgehend von N-Chlorphthalimid und Dicyclohexyldisulfid N-(Cyclohexylthio)-phthalimid hergestellt werden kann. Jedoch sind bei diesem Verfahren die Ausbeuten mit 60 bzw. 67 % nicht befriedigend.

Nach einem Verfahren der US-Patentschrift 3 579 460 werden Phthalimid und eine Lösung von Cyclohexylsulfenylchlorid in Dimethylformamid zusammengebracht. Dazu wird eine äquimolare Menge eines tertiären Amins gegeben und das entstandene Reaktionsprodukt durch Wasserzugabe ausgefällt. Nachteile dieses Verfahrens sind die Verwendung von Dimethylformamid, das nur schwer wieder zurückgewonnen werden kann, die großen Mengen Wasser, die nötig sind, um das N-(Cyclohexylthio)-phthalimid aus dem organischen Lösungsmittel zu isolieren und die Entfernung und Rückgewinnung des tertiären Amins.

Ähnliches gilt für das Verfahren von M. Behforouz und J. B. Kerwood in J. Org. Chem. *34,* 51-55 (1969), das in $CCl_4$ in Gegenwart eines tertiären Amins durchgeführt wird. Im übrigen ist dieser Literaturstelle zu entnehmen, daß N-(Cyclohexylthio)-phthalimid in alkalischen Lösungen instabil ist und schnell hydrolisiert.

Weiterhin ist allgemein bekannt (Houben-Weyl, Methoden der organischen Chemie Band *IX,* Seite 275 ff), daß sich Alkylsulfenylchloride in Wasser insbesondere in wäßrigem Alkalihydroxid sehr schnell zersetzen.

Umsetzungen von Phthalimid und Sulfenylchloriden in wäßrigem Alkali sind aus der österreichischen Patentschrift 226 680 und aus der US Patentschrift 3 840 555 nur für den Fall bekannt, daß es sich um hoch- oder perhalogenierte Alkylsulfenylchloride handelt. Im Falle des N-(2-Fluor-1,1,2,2-tetrachlorethylthio)-phthalimid beträgt die Ausbeute 77 % d. Th.

Es war daher überraschend, daß es nach dem erfindungsgemäßen Verfahren gelang, N-(Cyclohexylthio)-phthalimid aus Phthalimid und Cyclohexylsulfenylchlorid in nahezu quantitativer Ausbeute herzustellen, indem als Base wäßrige Alkali- oder Erdalkalihydroxid-Lösungen eingesetzt werden.

Gegenstand der Erfindung ist somit ein Verfahren zur Herstellung von N-(Cyclohexylthio)-phthalimid durch Umsetzung von Phthalimid und Cyclohexylsulfenylchlorid in Gegenwart von Basen, das dadurch gekennzeichnet ist, daß als Base eine wäßrige Alkali- und/oder Erdalkalihydroxid-Lösung eingesetzt und die Reaktion in einem Temperaturbereich von − 10 bis 20 °C durchgeführt wird.

Als Base kommen Alkali- oder Erdalkalihydroxide gelöst oder aufgeschlämmt in Wasser zum Einsatz. Bevorzugt werden Natronlauge, Kalilauge oder Calciumhydroxid. Die Konzentration in Wasser ist beliebig. Die eingesetzte Base dient vorwiegend als Salzsäurefänger.

Zur Reaktion kann auch noch zusätzlich Wasser als Reaktionsmedium benutzt werden.

Das Wasser, sei es zusätzlich zugegeben oder als Lösungs- oder Suspensionsmittel für die Base benutzt, sollte mindestens in solchen Mengen anwesend sein, daß das während der Reaktion sich bildende Alkali- oder Erdalkalichlorid gelöst ist. Die obere Mengenbegrenzung ist an sich beliebig. Aus wirtschaftlichen Gründen empfiehlt es sich jedoch nicht mehr Wasser zuzugeben, als erforderlich ist, um das aus Phthalimid und Base sich bildende Phthalimidsalz lösen zu können. Das Phthalimid kann jedoch ohne Nachteil auch als Suspension vorliegen.

Cyclohexylsulfenylchlorid kann nach mehreren Verfahren hergestellt werden, bevorzugt jedoch aus Cyclohexylmercaptan oder Dicyclohexyldisulfid und Chlor. Das Cyclohexylsulfenylchlorid kann in einem organischen Lösungsmittel vorliegen oder in Substanz.

Um eine bessere Durchmischung der wäßrigen und organischen Bestandteile bei der Reaktion zu erzielen, können Emulgatoren, die im Alkalischen wirken, zugesetzt werden.

Phthalimid, Cyclohexylsulfenylchlorid und die Base werden bevorzugt im gleichen molaren Verhältnis umgesetzt. Phthalimid und die Base können aber auch, bezogen auf das Sulfenylchlorid, in einem molaren Überschuß eingesetzt werden. Sollte ein Überschuß in Betracht kommen, empfiehlt es sich 1-10 Mol-% nicht zu überschreiten.

Die Reaktion verläuft nach folgenden Schema :

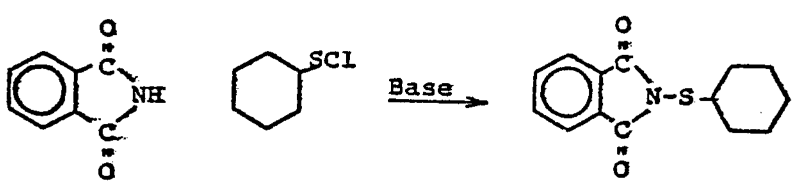

Wird die Reaktion bei Temperaturen unter 0 °C durchgeführt, so gibt man schmelzpunkterniedrigende Substanzen wie Salze oder wasserlösliche Lösungsmittel zu.

Die erfindungsgemäß hergestellte Verbindung wird zur Verzögerung der Anvulkanisation von natürlichen und/oder synthetischen Kautschuken verwendet.

## Beispiel 1

In einem Rührgefäß werden vorgelegt :

200 Gew.-Teile Wasser
29,4 Gew.-Teile Phthalimid
0,1 Gew.-Teile Emulgator Erkantol BXG

Das Gemisch wird auf 5 °C abgekühlt. Dann läßt man unter Rühren zulaufen :

18 Gew.-Teile NaOH 45 %ig
30,1 Gew.-Teile Cyclohexylsulfenylchlorid in
100 Gew.-Teilen Pentan

Nach 15 Minuten Nachrührzeit wird das ausgefallene feste Produkt von den flüssigen Phasen getrennt, einmal mit 20 Volumenteilen Pentan, danach mit Wasser gewaschen und das Produkt getrocknet.

Ausbeute : Gew.-Teile N-(Cyclohexylthio)-phthalimid ≙ 97,9 % der Theorie, bezogen auf das eingesetzte Phthalimid bzw. Cyclohexylsulfenylchlorid

Der Gehalt an reiner Substanz beträgt 98 %.

## Beispiel 2

In einem Rührgefäß werden vorgelegt :

100 Gew.-Teile Wasser
29,4 Gew.-Teile Phthalimid
0,1 Gew.-Teile Emulgator Erkantol BXG

Das Gemisch wird auf 5 °C abgekühlt. Dann läßt man unter Rühren zulaufen :

39,4 Gew.-Teile Kalilauge 50 %ig
30,1 Gew.-Teile Cyclohexylsulfenylchlorid in
120 Gew.-Teile Hexan

Nach 15 Minuten Nachrührzeit wird das ausgefallene feste Produkt von den flüssigen Phasen getrennt, einmal mit 20 Volumenteilen Hexan, danach mit Wasser gewaschen und das Produkt getrocknet.

Ausbeute : 50,5 Gew.-Teile N-(Cyclohexylthio)-phthalimid ≙ 96,7 % der Theorie, bezogen auf das eingesetzte Phthalimid bzw. Cyclohexylsulfenylchlorid.

Der Gehalt an reiner Substanz beträgt 98 %.

## Beispiel 3

In einem Rührgefäß werden vorgelegt :

100 Gew.-Teile Wasser
29,4 Gew.-Teile Phthalimid
0,1 Gew.-Teile Emulgator Erkantol BXG

Das Gemisch wird auf 10 °C abgekühlt. Dann läßt man unter Rühren zulaufen :

130 Gew.-Teile Kalkmilch 10 %ig
30,1 Gew.-Teile Cyclohexylsulfenylchlorid in
400 Gew.-Teile Toluol

Nach 15 Minuten Nachrührzeit werden die beiden Phasen getrennt, die organische getrocknet und auf den 10. Teilen eingeengt. Dazu gibt man 100 Gew.-Teile Pentan und trennt das feste Produkt ab, das getrocknet wird.

Ausbeute: 50 Gew.-Teile N-(Cyclohexylthio)-phthalimid ≙ 95,8 % der Theorie, bezogen auf den eingesetzte Phthalimid bzw. Cyclohexylsulfenylchlorid.

Der Gehalt an reiner Substanz beträgt 99 %.

**Ansprüche**

1. Verfahren zur Herstellung von N-(Cyclohexylthio)-phthalimid durch Umsetzung von Phthalimid mit Cyclohexylsulfenylchlorid in Gegenwart einer Base, dadurch gekennzeichnet, daß als Base eine wässrige Alkali- oder Erdalkalihydroxid-Lösung eingesetzt und die Reaktion in einem Temperaturbereich von − 10 bis 20 °C durchgeführt wird.

2. Verfahren nach Anspruch 1, dadurch gekennzeichnet, daß Phthalimid, Cyclohexylsulfenylchlorid und die Base in gleichem molarem Mengenverhältnis umgesetzt wird.

3. Verfahren nach Anspruch 1, dadurch gekennzeichnet, daß Phthalimid und die Base im 1-10 Mol %igem Überschuß, bezogen auf Cyclohexylsulfenylchlorid umgesetzt wird.

4. Verfahren nach Ansprüchen 1 bis 3, dadurch gekennzeichnet, daß zusätzlich Emulgatoren eingesetzt werden, die im Alkalischen wirken.

**Claims**

1. Process for the preparation of N-(Cyclohexylthio)-phthalimide by reacting phthalimide with cyclohexylsulphenylchloride in the presence of a base, characterised in that an aqueous alkali or alkaline earth metal hydroxide solution is used as the base and the reaction is carried out in a temperature range of − 10 to 20 °C.

2. Process according to Claim 1, characterised in that phthalimide, cyclohexylsulphenylchloride and the base are reacted in an equal molar proportion.

3. Process according to Claim 1, characterised in that phthalimide and the base are reacted in a 1-10 mol% excess, based on cyclohexylsulphenylchloride.

4. Process according to Claims 1 to 3, characterised in that emulsifiers which act in an alkaline medium are additionally used.

**Revendications**

1. Procédé de fabrication de la N-(cyclohexylthio)-phtalimide par réaction de la phtalimide avec du chlorure de cyclohexylsulfényle en présence d'une base, caractérisé en ce qu'on utilise comme base une solution aqueuse d'hydroxyde alcalin ou alcalino-terreux et en ce qu'on exécute la réaction dans un intervalle de température de −10 à 20 °C.

2. Procédé selon la revendication 1, caractérisé en ce qu'on fait réagir la phtalimide, le chlorure de cyclohexylsulfényle et la base dans le même rapport quantitatif molaire.

3. Procédé selon la revendication 1, caractérisé en ce qu'on fait réagir la phtalimide et la base en un excès de 1 à 10 moles% par rapport au chlorure de cyclohexylsulfényle.

4. Procédé selon les revendications 1 à 3, caractérisé en ce qu'on utilise supplémentairement des émulsifiants qui sont actifs en milieu alcalin.